# EUROPEAN PATENT APPLICATION

(11) **EP 1 541 181 A1**
(43) Date of publication of application: **15.06.2005**
(21) Application number: 04257461.6
(22) Date of filing: 01.12.2004
(51) Int. Cl.: A61L 17/00, A61B 17/04

(54) **Braided hollow suture**

(30) Priority: 02.12.2003 US 526665 P; 23.02.2004 US 785773
(71) Applicant: Arthrotek, Inc., Warsaw, Indiana 46581 (US)
(72) Inventor: Walters, Troy M., Plymouth Indiana 46563 (US); Stone, Kevin T., Winona Lake Indiana 46590 (US); Clarke, Steven R., Mansfield Massachusetts 02048 (US)
(74) Representative: Giles, Ashley Simon

(57) **Abstract**

A suture for a suture anchor or surgical needle. The suture is a tubular sheath defining an empty bore and is braided using biocompatible filaments of the same or different materials. The sheath has a flattened annular cross-section.

## Description

### CROSS-REFERENCE TO RELATED APPLICATIONS

This application claims the benefit of U.S. Provisional Application No. 60/526,665, filed on December 2, 2003. The disclosure of the above application is incorporated herein by reference.

### INTRODUCTION

Surgical sutures are available in a variety of materials in monofilament or multifilament forms, including braided structures. Braided sutures can be, for example, solid, or spiroid or include a cover surrounding a core. To maintain their strength, such sutures can be stiff and difficult to manipulate during surgical procedures, especially during those procedures that involve minimally invasive surgery. Cylindrically shaped sutures may also be difficult to knot or to retain a knot due to the type of suture material that is employed.

Improved braided sutures that are strong, flexible, easy to knot and that retain a knot are still desirable.

### SUMMARY

The present teachings provide a suture that can be used with a suture anchor and/or a surgical needle. The suture has a tubular braided sheath having a flattened annular cross-section defining an empty bore. The sheath is braided using biocompatible fibers of the same or different materials.

The present teachings also provide a method of making a suture. The method includes providing a mandrel, braiding pluralities of first and second filaments around the mandrel to form a sheath, and removing the mandrel.

The present teaching provide a method of suturing. The method includes providing a flattened tubular braided suture, loading the suture to a suture anchor; implanting the suture anchor; and tying a suture knot.

Further areas of applicability of the present invention will become apparent from the detailed description provided hereinafter. It should be understood that the detailed description and specific examples are intended for purposes of illustration only and are not intended to limit the scope of the invention.

### BRIEF DESCRIPTION OF THE DRAWINGS

The present invention will become more fully understood from the detailed description and the accompanying drawings, wherein:

FIG. 1 is an environmental isometric view of a suture according to the present teachings, shown in the process of braiding;

FIG. 2 is an isometric view of a finished suture according to the present teachings;

FIG. 3 is a schematic diagram of a braiding pattern according to the present teachings;

FIG. 4 illustrates a suture attached to an anchor, according to the present teachings;

FIG. 5A illustrates a suture threaded to a needle, according to the present teachings;

FIG. 5B a cross-sectional view of a suture swaged to a needle, according to the present teachings;

FIG. 6 illustrates forming a knot with a suture according to the present teachings; and

FIG. 7 illustrates the knot of FIG. 6 in the process of being tightened.

### DETAILED DESCRIPTION OF VARIOUS EMBODIMENTS

The following description of the various embodiments is merely exemplary in nature and is in no way intended to limit the invention, its application, or uses.

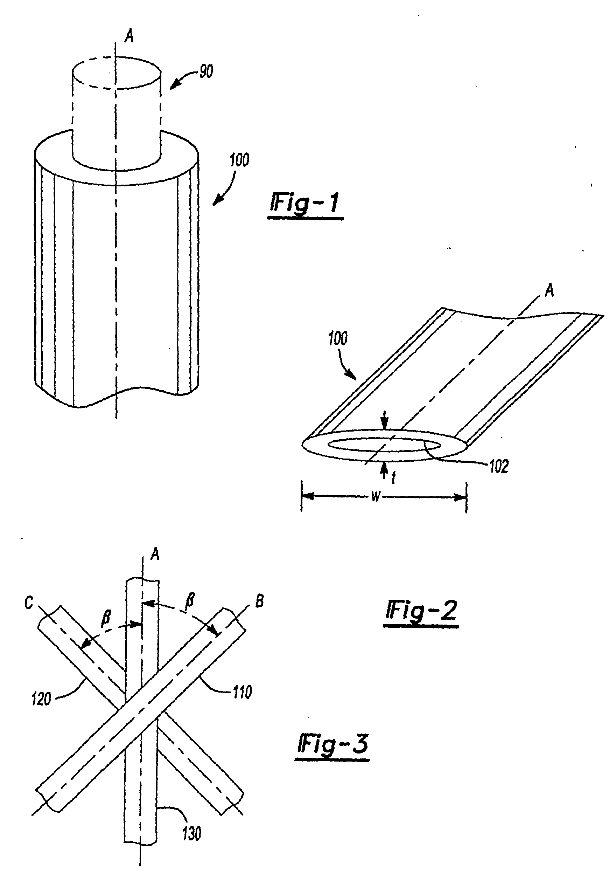

Referring to FIGS. 1 and 2, a suture 100 for use in any medical applications, such as orthopaedic applications, can be made as a flattened tubular sheath that defines a hollow bore 102. The suture 100 has a longitudinal axis "A" and can be braided as a three-dimensional structure around a mandrel 90, which is removed after braiding, leaving the empty bore 102. Removal of the mandrel 90 causes the suture 100 to take a flattened sleeve-like shape with an annular cross-section, as shown in FIG. 2. The finished suture 100 contains no core material and is flexible. The width "w" of the suture 100 can be about twice the thickness "t" of the suture 100.

Referring to FIGS. 1 and 3, the suture 100 can be braided around the mandrel 90 using a plurality of first filaments 110 oriented along an axis "B" and a plurality of second filaments 120 oriented along an axis "C". The first and second filaments 110, 120 are spirally braided around the mandrel 90 and are oppositely (counterclockwise and clockwise) spirally inclined relative to the axis A at angles β, for example 45° or any other angle, resulting in a biaxial braid. The suture 100 can also be braided in a triaxial pattern including a plurality of axial filaments 130, which are interlaced with the first and second filaments 110, 120. The axial filaments 130 retain their straight orientation parallel to the direction of the axis A. Although the braiding structure of FIG. 3 is shown to be symmetric and balanced about the axis A, the suture need not be thus limited.

Depending on the size of the suture 100 and the size of the filaments selected, two, four, eight, etc., of each of the first, second and axial filaments can be used to form the braided structure. For example, eight first filaments having a denier of about 120, eight second filaments having a denier of about 120, and eight axial filaments having a denier of about 120, can be used to obtained a suture 100 with width of about 0.045 inches and thickness of about 0.015 inches, for a diameter or a USP (United States Pharmacopoeia) size of 3. Of course, any size or denier is contemplated herein. The braided structure itself can be of any pattern, such as "one filament over and one filament under", "two filaments over and two filaments under" and so on. The braiding itself can be done using commercially available machines.

The first, second, and axial filaments 110, 120, 130 can be made from the same or different materials. The materials of the first, second and axial filaments 110, 120, 130 can be selected in any combination from a group of biocompatible, bioabsorbable or non-bioabsorbable, and natural or artificial materials, depending on the particular application. The filaments 110, 120, 130 can be made for example from ultra high molecular weight polyethylene, nylon, polyesters, polyamides, polyolefins, fluorocopolymers, cotton, linen, silk, to name but a few. Filaments of ultra high molecular weight polyethylene filaments are commercially available from DSM under the tradename Dyneema. As an example, the first and second filaments 110, 120 can be made of the same or different polyester materials, including high tenacity polyester materials, and the axial filaments 130 can be made of ultra high molecular weight polyethylene that has high tenacity and low elongation. This type of combination results in a flattened suture 100 which is flexible, smooth, easy to tie in knots, and yet maintains its axial strength, length, and its shape by the reinforcing action of low elongation/high tenacity axial filaments 130. In this regard, the axial filaments 130 prevent the suture 100 from elongating and shrinking in width and thickness upon applying a tension along the axis A.

Any of the first, second and axial filaments, 110, 120, 130 and or the suture 100 itself can be coated with natural or artificial coatings to improve lubricity. Any of the filaments can also be provided with color for coding the suture 100 or for improving its visibility during the procedure. For example, a single filament can be a colored fiber that will impart color to the suture 100 when the other fibers are translucent or will provide contrasting color when the other fibers are of other opaque color or colors. Alternatively, the entire suture 100 can be dyed in a single color.

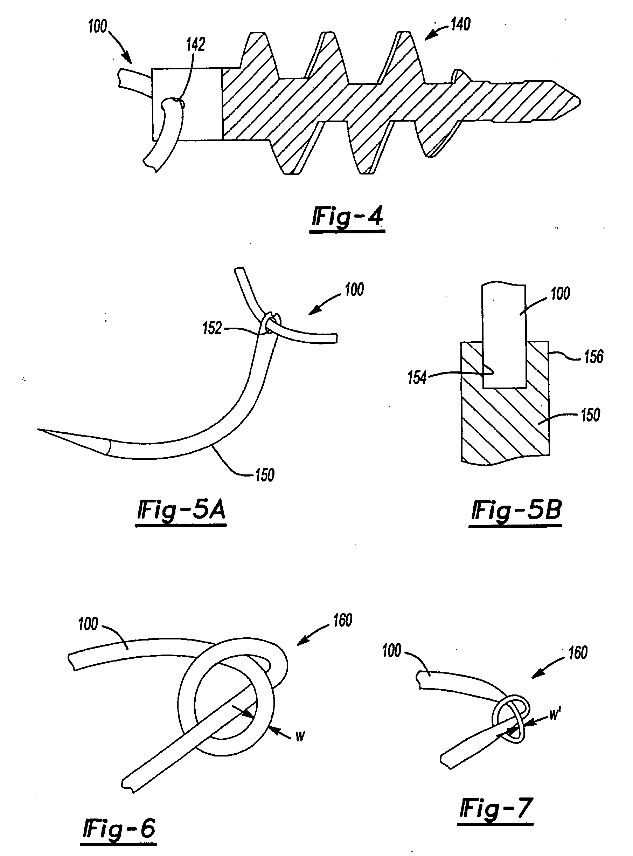

The suture 100 can be used in combination with a suture device including suture anchors, surgical needles, suture passers, suture retrievers, suture management systems, and/or any devices that can pass, pull or push suture through tissue. The suture 100 can be used, for example, with the exemplary suture anchor 140 shown in FIG. 4, in which the suture 100 is threaded through an eyelet 142 of the anchor 140. The suture 100 can also be used with any type of other similar fixation devices, including screws, pins, nails, etc., and also with a surgical needle 150 having an eyelet 152, as shown in FIG. 5A, or with a surgical needle 150 having a bore 154 into which the suture 100 is inserted and then retained by swaging or crimping the lateral surface 156 of the bore 154, as shown in FIG. 5B. To accommodate the flatness of the suture 100, as defined by the ratio of width w over thickness t, the eyelet 142 of the anchor 140 and the eyelet 152 of the needle may be modified to take a flatter, elongated shape. The flatter shape of the suture 100 reduces the wear and tear of the suture 100 by having a larger contact surface with the eyelets 142, 152, thereby distributing the load over a larger surface area. Similarly, when the suture is used tie ligaments and/or other soft tissue to bone, the load is distributed over a larger area of soft tissue reducing impinging, tearing or cutting of the soft tissue. The cut ends of the suture 100 can be coated with a protective coating, such as an adhesive, to prevent fraying.

Referring to FIGS. 6 and 7, the flatness of the suture 100 contributes to its ability to tie knots 160 that do not back out. This is because when the knot 160 is tightened, the width of the suture 100, which is equal to w before the knot 160 is tightened in Fig. 6, is reduced to w', which is less than w, in the area of the knot 160. The wider width w outside the knot 160 prevents suture back-out and unknotting, as shown in FIG. 7. The width w' is reduced due to compressive forces in the area of the knot 160 as the suture 100 is compressed relative to the empty bore 102.

In operation, the suture 100 is threaded through the eyelet 142 of the suture anchor 140, such that the suture 100, facilitated by its hollow, ribbon-like shape, lays flat against the sides of the eyelet 142 for more even load distribution. The anchor 140 loaded with the suture 100 is implanted in a prepared surgical site or bone portion. The eyelet 142 can be elongated for better load distribution, although other eyelet shapes, such as circular or rounded can be used. In practice, the suture anchor 140 with the suture 100 loaded thereon may be implanted using an inserter, of the type known in the art (not shown). The suture 100 can also be threaded through the eyelet 152 or swaged or crimped into the bore 154 or preloaded to the surgical needle 150, to pass the suture 100 through soft tissue or to help knot the suture 100 after the suture anchor 140 is implanted. The eyelet 152 of the surgical needle 150 can also be elongated for better load distribution. After the inserter is removed, the free ends of the suture 100 can be tied to provide knots 160 that will not easily back out, because the flattened shape of the hollow suture 100 provides localized crimping or width reduction upon knotting, as shown in FIG. 7. The flattened shape of the suture 100 also distributes the load as the flattened hollow suture 100 passes through soft tissue to reduce or prevent tearing of the soft tissue.

The suture 100 can be used in various orthopaedic procedures, including microplasty procedures that involve small incisions and minimally invasive surgery. Examples include, ACL/PCL reconstruction, hip and shoulder replacement, tendon repair, etc.

The description of the invention is merely exemplary in nature and, thus, variations that do not depart from the gist of the invention are intended to be within the scope of the invention. Such variations are not to be regarded as a departure from the spirit and scope of the invention.

## Claims

1. A suture comprising a tubular braided sheath having a flattened annular cross-section defining an empty bore.

2. The suture of claim 1, further comprising pluralities of first and second filaments oppositely spirally inclined relative to a longitudinal axis of the sheath.

3. The suture of claim 2, wherein the first and second filaments comprise different materials.

4. The suture of claim 2, wherein the first and second filaments are made of biocompatible materials.

5. The suture of claim 2, wherein the first and second filaments are selected from the group consisting of polyethylene, nylon, polyesters, polyamides, polyolefins, and fluorocopolymers.

6. The suture of claim 2, further comprising a plurality of axial filaments interwoven with the first and second filaments.

7. The suture of claim 6, wherein at least one filament is of contrasting color.

8. The suture of claim 6, wherein the first, second and axial filaments comprise polyester.

9. The suture of claim 6, wherein the axial filaments are of high tenacity and low elongation.

10. The suture of claim 9, wherein the axial filaments comprise ultra high molecular weight polyethylene.

11. The suture of claim 10, wherein the first and second filaments comprise ultra high molecular weight polyethylene.

12. The suture of claim 9, wherein the first and second filaments comprise polyester.

13. The suture of claim 2, further comprising a lubricity coating.

14. The suture of claim 2, wherein each filament comprises a lubricity coating.

15. The suture of claim 1 in combination with a surgical needle.

16. The suture of claim 1 in combination with a suture anchor.

17. The combination of claim 16, wherein the suture anchor has an elongated eyelet providing contact and load distribution along a width of the suture.

18. The suture of claim 1, wherein the sheath has a width to thickness ratio greater than 1.

19. The suture of claim 18, wherein the width to thickness ratio reduces knot back-out.

20. The suture of claim 18, wherein the width to thickness ratio reduces soft tissue damage.

21. The suture of claim 1, wherein the sheath has a width to thickness ratio greater than 1.5.

22. The suture of claim 1, wherein the sheath has a width to thickness ratio greater than 2.

23. A method of making a suture comprising:
providing a mandrel;
braiding pluralities of first and second filaments around the mandrel to form a sheath; and
removing the mandrel to form an empty bore.

24. The method of claim 23, further comprising braiding a plurality of axial filaments with the first and second filaments.

25. A method of suturing comprising:
providing a flattened tubular braided suture;
loading the suture to a suture device;
deploying the suture device; and
tying a suture knot.

26. The method of claim 25, wherein providing a braided suture includes providing a suture having pluralities of first and second filaments oppositely spirally inclined relative to a longitudinal axis of the suture, and a plurality of axial filaments interwoven with the first and second filaments.

27. The method of claim 25, wherein the suture device is a suture anchor.

28. The method of claim 25, wherein the suture device is a surgical needle.

29. The method of claim 25, wherein tying a suture knot comprises compressing a width of the suture inside the knot such that the width of the suture is narrower inside the knot that outside the knot.

30. The method of claim 25, wherein deploying the suture device comprises passing the suture through tissue using the suture device.
